Europäisches Patentamt

European Patent Office

Office européen des brevets

(11) Veröffentlichungsnummer: **0 337 092**
**A2**

## (12) EUROPÄISCHE PATENTANMELDUNG

(21) Anmeldenummer: **89103438.1**

(51) Int. Cl.⁴: **G01P 5/14 , A61M 5/14**

(22) Anmeldetag: **28.02.89**

(30) Priorität: **09.04.88 DE 8804698 U**

(43) Veröffentlichungstag der Anmeldung:
**18.10.89 Patentblatt 89/42**

(84) Benannte Vertragsstaaten:
**AT BE CH DE ES FR GB IT LI NL**

(71) Anmelder: **Labionics Aktiengesellschaft**
**Ziegelbrückenstrasse 22a**
**CH-8867 Niederurnen(CH)**

(72) Erfinder: **Hagen, Dieter**
**Lottbekheide 17**
**D-2000 Hamburg 65(DE)**

(74) Vertreter: **Dipl.-Ing. H. Hauck Dipl.-Phys. W.**
**Schmitz Dipl.-Ing. E. Graalfs Dipl.-Ing. W.**
**Wehnert Dr.-Ing. W. Döring**
**Neuer Wall 41**
**D-2000 Hamburg 36(DE)**

(54) **Durchflussmessvorrichtung für Fluide.**

(57) Durchflußmeßvorrichtung für Fluide mit einer Meßstrecke und einem Meßwandler zwischen einem Flüssigkeitszu- und -ablauf, wobei die Meßstrecke mindestens zwei Druckkammern (11,12) aufweist, die jeweils über einen kalibrierten Durchgang (15) miteinander verbunden sind und jeder Druckkammer (11, 12) ein Meßwandler (30) zugeordnet ist zur Erzeugung eines dem Druck in der Druckkammer (11, 12) entsprechenden Meßsignals.

FIG.1

EP 0 337 092 A2

## Durchflußmeßvorrichtung für Fluide

Die Erfindung betrifft eine Durchflußmeßvorrichtung für Fluide oder dergleichen nach dem Oberbegriff des Anspruchs 1.

Das Zumessen von Infusionsflüssigkeiten erfolgt konventionell über eine steuerbare Infusionspumpe, die naturgemäß nur eine indirekte Erfassung des geförderten Volumens ermöglicht. Bekannte Infusionsvorrichtungen arbeiten auch nach dem Prinzip eines Infusionstropfes; sie enthalten Tropfensensoren, die beispielsweise fotoelektrisch aufgebaut sind und die Anwesenheit eines Tropfens registrieren und ein Anwesenheitssignal an eine Regel-, Auswerte-oder Überwachungseinrichtung abgeben. Diese ermittelt durch Auf integrieren der Tropfen den Durchfluß. Die Tropfen werden im Tropfensensor selbst erzeugt und mittels Schwerkraft an dem Sensor vorbeigeleitet, der in einem Auffanggefäß des Tropfensensors angeordnet ist.

Neben dem Nachteil, daß Tropfensensoren eine vertikale Einbaulage fordern, haben sie grundsätzlich auch den Nachteil, daß allein durch Zählen der Tropfen der Durchsatz infolge unterschiedlicher Tropfengrößen nicht exakt bestimmt werden kann. Es handelt sich hierbei also um eine angenäherte Durchflußbestimmung. Die Tropfensensoren haben den weiteren Nachteil, daß unter Umständen hohe Meßtotzeiten infolge der Tropfenfrequenz auftreten, besonders bei einem Durchsatz von nur einigen Millimetern pro Stunde. Ferner bilden der oftmals empfindliche Sensor und die Tropfenstrecke eine untrennbare Einheit, zumindest aber eine Einheit, bei der der Sensor mit der zu messenden durchfließenden Flüssigkeit in Berühung kommt. Es ist daher vor jedem neuen Einsatz eine aufwendige Sterilisation erforderlich.

Aus der DE-PS 29 44 186 ist eine weitere Möglichkeit bekannt, zum Beispiel bei Infusionsgeräten den Infusionsflüssigkeitsdurchsatz genau zu bestimmen. Bei dieser vorgenannten Art wird die Tatsache ausgenutzt, daß, falls nicht andere Fehler auftreten, der Verstellweg bzw. die Drehzahl einer volumetrischen Pumpe direkt proportional der abgegebenen Fördermenge ist, so daß auf diese Weise ein fehlerfreier Betrieb mittels einer entsprechenden Regelung sichergestellt werden kann. Hierbei ist eine Pumpe mit einer Rasterscheibe gekoppelt, die mit Hilfe einer Abtastvorrichtung abgetastet wird. Die Abtastvorrichtung erzeugt dann ein Signal, dessen Größe abhängig ist von der Drehzahl der Rasterscheibe. Dieses Signal wird auf einen Steuereingang eines Mikroprozessors gegeben. Mit Hilfe der Rasterscheibe und der Abtastvorrichtung kann dann ein Ist-Signal gebildet werden, daß im Mikroprozessor mit einem Soll-Signal für die Förderleistung verglichen wird. Eine Regelabweichung führt dann zu einer entsprechenden Veränderung der Motordrehzahl, beispielsweise eines Schrittmotors. Bei dieser Regeleinrichtung wird das tatsächliche Durchflußvolumen also indirekt über die Motordrehzahl bestimmt. Wegen anderer möglicher Fehlerquellen ist bei dieser Regeleinrichtung noch ein Tropfensensor vorgesehen, der ebenfalls sein Signal zum Mikroprozessor abgibt, welcher bei Differenzen zwischen diesem Ist-Signal und dem Ist-Signal der Rasterscheibe eine Fehleranzeige veranlaßt.

Durchflußmesser für flüssige oder gasförmige Fluide benötigen üblicherweise einen in der Leitung angeordneten Rotor, dessen Drehzahl bzw. Drehgeschwindigkeit von der Durchströmmenge abhängt. Die Welle des Rotors ist über die Wandung der Leitung nach außen geführt zur Erzeugung eines von der Wellendrehung abhängigen Meßsignals. Ist das zu messende Fluid chemisch aggressiv, giftig oder radioaktiv, müssen außerordentlich gute Dichtungen für die Wellendurchführung verwendet werden. Außerdem gelangt der Rotor ständig in Kontakt mit dem Fluid und muß nach seiner Benutzung gereinigt oder sterilisiert werden, was mit einem entsprechenden Aufwand verbunden ist. Schließlich ist es nahezu unmöglich, mit herkömmlichen Durchflußmeßvorrichtungen sehr geringe Strömungsmengen relativ genau zu ermitteln.

Der Erfindung liegt die Aufgabe zugrunde, eine Durchflußmeßvorrichtung für Fluide zu schaffen, die einen Kontakt mit dem Fluid nicht benötigt und unabhängig von ihrer Einbaulage auch sehr geringe Fluidmengen verhältnismäßig genau zu messen imstande ist.

Diese Aufgabe wird durch die Merkmale des Kennzeichnungsteils des Anspruchs 1 gelöst.

Die erfindungsgemäße Durchflußmeßvorrichtung sieht im Zuge des Strömungsweges mindestens zwei Druckkammern vor, die über einen kalibrierten Durchgang miteinander verbunden sind. Jeder Druckkammer ist ein Meßwandler zugeordnet, der den Druck in der Druckkammer mißt. Die erfindungsgemäße Durchflußmeßvorrichtung geht von der bekannten Beziehung aus, daß die Strömungsmenge ermittelt werden kann, wenn der Druck in den beiden Druckkammern gemessen wird und der Durchgang zwischen den Druckkammern eine bekannte Geometrie besitzt. Die Formel lautet:

$$\text{Durchflußmenge} = (\pi\, \Delta\, PR^4)/(8\,\gamma l)$$

wobei $\Delta P$ die Druckdifferenz der Drücke in den Druckkammern ist, R der Radius des Durchgangs, $\gamma$ die kinematische Viskosität des Fluids und l die Länge des Durchgangs.

Der Durchgang kann von einer Lochblende gebildet sein. Alternativ kann ein kanalförmiger Durchgang vorgesehen werden in Form eines Röhrchens oder dergleichen.

Es sind verschiedene konstruktive Möglichkeiten denkbar, den Druck in den Druckkammern mit Hilfe eines Drucksensors zu messen. Eine Ausgestaltung der Erfindung sieht vor, daß die Druckkammern einen nachgebenden Wandabschnitt aufweisen und der Meßwandler dem nachgebenden Wandabschnitt zugeordnet ist zur Messung der Auslenkung des Wandabschnitts. Der nachgebende Wandabschnitt kann in sich starr, jedoch beweglich gelagert sein. Alternativ kann nach einer Ausgestaltung der Erfindung der Wandabschnitt von einer vorzugsweise dünnen Membrane gebildet sein, die im unbelasteten Zustand im wesentlichen flach ist. Beim Aufbau des Drucks in der Druckkammer wird die Membrane nach außen gewölbt. Die Auslenkung des Wandabschnitts bzw. der Membrane kann mit Hilfe geeigneter Drucksensoren erfaßt werden.

Die Ausbildung eines die Auslenkung des nachgebenden Wandabschnitts ermittelten Meßwandlers läßt verschiedene Möglichkeiten zu. Eine besteht erfindungsgemäß darin, daß der Meßwandler ebenfalls einen nachgiebigen Wandabschnitt aufweist, vorzugsweise eine Meßmembrane, die mit der Membrane bzw. dem nachgiebigen Wandabschnitt einer Druckkammer in Kontakt steht und die ihrerseits in Wirkkontakt mit der Ansprechfläche des Meßwandlers bzw. einer Druckmeßvorrichtung steht.

Ein besonders vorteilhafter Aufbau für den Meßwandler besteht erfindungsgemäß darin, daß der nachgebende Wandab schnitt bzw. die Meßmembrane einen von unnachgiebigen Wänden gebildeten Hohlraum des Meßwandlers abschließt, dem die Ansprechfläche des Meßwandlers bzw. der Druckmeßvorrichtung zugeordnet ist. Eine Lageveränderung der Meßmembrane bzw. des nachgiebigen Wandabschnitts führt zu einer Druckveränderung des Fluids im abgeschlossenen Hohlraum, die sich an der Ansprech- oder Wirkfläche des Meßwandlers bzw. der Druckmeßvorrichtung bemerkbar macht. Vorzugsweise ist der Hohlraum mit einer Meßflüssigkeit gefüllt. Aufgrund der Imkompressibilität der Flüssigkeit wird eine Auslenkung des Wandabschnitts bzw. der Meßmembrane direkt auf die Ansprechfläche des Meßwandlers übertragen. Die Meßmembrane ist bei Nichtkontakt mit dem nachgebenden Wandabschnitt der Druckkammer vorzugsweise unter Spannung nach außen gewölbt. Beim Aufsetzen auf den nachgiebigen Wandabschnitt wird die Meßmembrane zurückverformt, wobei jedoch im Fall einer Meßflüssigkeit im Hohlraum eine Ausweichmöglichkeit geschaffen werden muß. Diese besteht zum Beispiel in einem Ausgleichsbehälter, vorzugsweise in Form eines elastischen Ballons, der über eine einen Absperrhahn aufweisende Verbindungsleitung mit dem Hohlraum verbunden ist.

Bei einer alternativen Ausgestaltung der Erfindung sind die zu den Druckkammern gehörenden Membranen aus dünnem elastischem sich piezoelektrisch verhaltendem Material gebildet zur Erzeugung eines der Auslenkung der Membran entsprechenden Signals.

Mit Hilfe der erfindungsgemäßen Durchflußmeßvorrichtung kann eine Mengenmessung von sehr geringen Werten, zum Beispiel 0,1 ml/h bis zu einigen l/s mit der gleichen Anordnung gemessen werden. Bei größeren Strömungsmengen läßt sich eine Messung zwar nach dem gleichen Prinzip durchführen, sie erfordert jedoch eine etwas abgewandelte Vorrichtung.

Besonders vorteilhaft bei der erfindungsgemäßen Vorrichtung ist die Tatsache, daß zwischen den mit dem Fluid in Kontakt kommenden Teilen der Vorrichtung und dem Meßwandler bzw. der Druckmeßvorrichtung keine unmittelbare Verbindung besteht; die Druckkammern mit dem Verbindungsdurchgang lassen sich in Form einer Zelle ausbilden, die beispielsweise aus Kunststoffmaterial geformt ist. Sie kann nach dem Betrieb ohne weiteres fortgeworfen und durch eine andere ersetzt werden, während die relativ aufwendige Meßeinrichtung im Einsatz bleiben kann und auch nicht gereinigt oder sterilisiert werden muß.

Ein Hauptanwendungsgebiet der erfindungsgemäßen Durchflußmeßvorrichtung erstreckt sich auf die Steueranordnungen für medizinische Infusionspumpen. Sie kann jedoch auch für andere Dosierpumpen eingesetzt werden, insbesondere bei solchen, die gefährliche Fluide, wie giftige, chemisch aggressive, radioaktive oder dergleichen Fluide fördern. In jedem Fall wird eine sichere, genaue und zuverlässige Durchflußmengenmessung ermöglicht, was vor allen Dingen bei der medizinischen Anwendung von großer Bedeutung ist. Die erfindungsgemäße Vorrichtung kann unabhängig von der Infusionspumpe ausgebildet sein und daher in Verbindung mit herkömmlichen Infusionspumpen eingesetzt werden. Die erfindungsgemäße Vorrichtung kann jedoch auch mit einer angepaßten Infusionspumpe versehen werden. Hierauf wird weiter unten noch näher eingegangen.

Wie bereits ausgeführt, kann die erfindungsgemäße Vorrichtung auch mit mehr als zwei Druckkammern versehen werden. Indem die Druckdifferenz zwischen mehr als zwei Druckkammern gemessen wird, können Unregelmäßigkeiten sehr leicht festgestellt werden, die zum Beispiel darin bestehen, daß nicht alle Drucksensoren zuverlässig arbeiten oder die Auswertevorrichtung einen Fehler enthält.

Es versteht sich, daß die Druckkammern so ausgebildet sind, insbesondere an ihrem Auslaßende, daß unabhängig von der Lage der Meßvorrichtung die Ansammlung von Luftbläschen vermieden wird, wodurch sonst eine Fehlmessung verursacht würde.

In einer weiteren Ausgestaltung der Erfindung ist vorgesehen, daß der in Strömungsrichtung gesehen ersten Druckkammer eine Vorkammer vorgeordnet ist, deren Volumen größer ist als das gesamte Volumen der Druckkammer und der nachgeschalteten Ausgangsleitung hinter der letzten Druckkammer, dem Einlaß der Vorkammer eine Verschlußvorrichtung und der Vorkammer eine das Arbeitsvolumen der Vorkammer verkleinernde Pumpvorrichtung zugeordnet ist. Bei der Anwendung in einem Infusionssystem wird üblicherweise das Leitungssystem mit Infusionsflüssigkeit gefüllt, bevor ein Anschluß an die Infusionspumpe erfolgt. Da der Durchmesser des kalibrierten Durchgangs verhältnismäßig klein ist und die Flüssigkeit normalerweise durch Schwerkraft einströmt, würde es eine verhältnismäßig große Zeit erfordern, bis das gesamte Leitungssystem gefüllt ist. Zu diesem Zweck ist die Vorkammer vorgesehen. Diese kann in üblicher Weise durch Schwerkraft relativ rasch mit Flüssigkeit gefüllt werden. Anschließend wird die Vorkammer geschlossen und zum Beispiel durch einen Druck auf die vorzugsweise mit elastischer Wand versehene Vorkammer die Flüssigkeit aus dieser heraus in die nachfolgenden Druckkammern und das anschließende Leitungssystem hineinbefördert. Anschließend kann das System in bekannter Weise an die Infusionspumpe angeschlossen werden. Es versteht sich, daß zum Beispiel ein Kolben zur Veränderung des Arbeitsvolumens der Vorkammer und zur Betätigung einer Klemme zum Abklemmen des Schlauchs vor der Vorkammer mit Hilfe geeigneter Betätigungsvorrichtungen automatisch betätigt werden kann, um einen schnellen und einwandfreien Betrieb des Infusionssystems zu gewährleisten.

Die Vorkammer kann nach einer weiteren Ausgestaltung der Erfindung auch durch einen elastomeren Stopfen oder ein Septum verschlossen sein. Die Flüssigkeit kann auf diese Weise mit Hilfe einer Injektionsspritze in die Vorkammer gefüllt werden, indem die Injektionsnadel durch das Septum gestochen wird. Ein zum Beispiel zur Verkleinerung des Volumens der Vorkammer vorgesehener Kolben drückt dann, wie oben beschrieben, die Flüssigkeit in das System. Auf diese Weise kann das System auch als Injektionspumpe verwendet werden, insbesondere als sogenannte vorzugsweise tragbare Mikropumpe. Der Kolben wird zum Beispiel mechanisch, elektromagnetisch, hydraulisch oder pneumatisch angetrieben und kann zum Entleeren der Vorkammer und somit zum Injizieren der Flüssigkeit zum Beispiel in den menschlichen Körper verwendet werden. Der Vorteil einer derartig aufgebauten Mikropumpe besteht gegenüber bisher bekannten Pumpen darin, daß die Flüssigkeit mit großer Genauigkeit und sehr gleichmäßig gefördert werden kann, insbesondere bei kleinen Flüssigkeitsmengen ab 0,1 ml/h.

Die Erfindung wird nachfolgend anhand von in Zeichnungen dargestellten Ausführungsbeispielen näher erläutert.

Fig. 1 zeigt eine Draufsicht auf eine schematisch dargestellte Meßstrecke der erfindungsgemäßen Durchflußmeßvorrichtung.

Fig. 2 zeigt eine Seitenansicht der Meßstrecke nach Fig. 1 mit zugeordneten Druckmeßwandlern.

Fig. 3 zeigt eine ähnliche Ansicht wie Fig. 2, jedoch nach dem Instellungbringen der Druckmeßwandler.

Fig. 4 zeigt eine Seitenansicht einer abgewandelten Meßstrecke der erfindungsgemäßen Vorrichtung.

Fig. 5 zeigt eine andere Seitenansicht eines Teils der Meßstrecke nach Fig. 4 mit Halterung und zusätzlicher Betätigungsvorrichtung.

Fig. 6 zeigt eine ähnliche Ansicht wie Fig. 4, jedoch mit geringfügiger Abwandlung.

Fig. 7 zeigt eine ähnliche Darstellung wie Fig. 5, jedoch als Seitenansicht von Fig. 6.

Bevor auf die in den Zeichnungen dargestellten Einzelheiten näher eingegangen wird, sei vorangestellt, daß jedes der beschriebenen Merkmale für sich oder in Verbindung mit Merkmalen der Ansprüche von erfindungswesentlicher Bedeutung sein kann. Ferner wird betont, daß die Zeichnungen äußerst schemahaft und nicht maßstäblich sind.

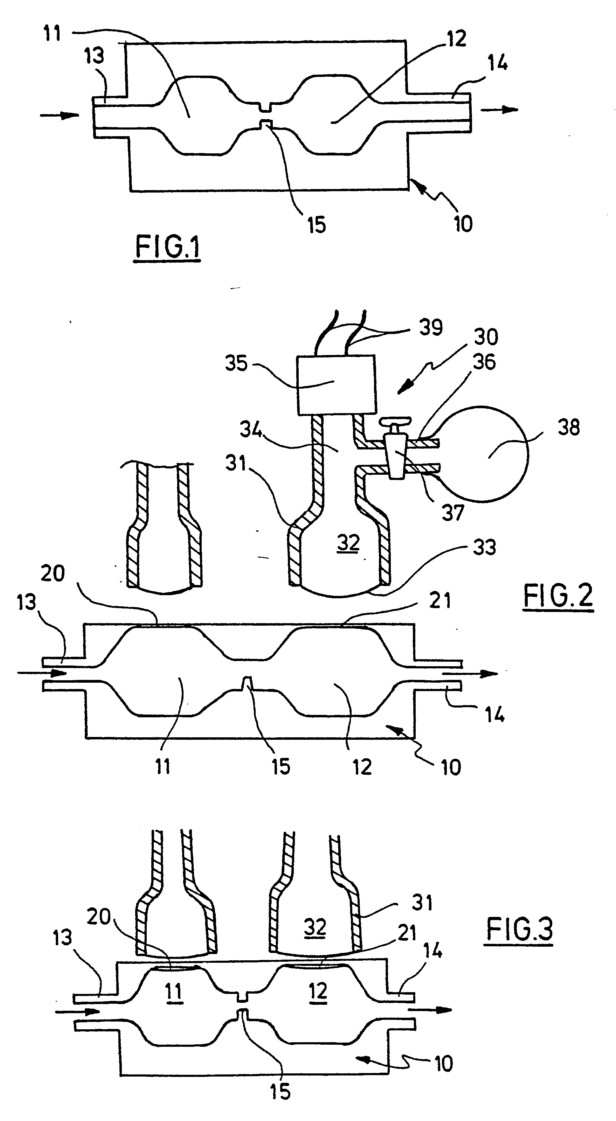

In Fig. 1 ist ein Kunststoffgehäuse 10 zu erkennen, in dem zwei Druckkammern 11, 12 geformt sind mit konkaven Wänden, die die Ansammlung von Luftbläschen insbesondere im Ausgangsbereich verhindern sollen. Die Druckkammer 11 ist mit einem Einlaßstutzen 13 und die Druckkammer 12 mit einem Auslaßstutzen 14 verbunden. Die Kammern 11, 12 sind über eine Lochblende 15 miteinander verbunden, deren Länge l und deren Radius R definiert sind. Das Gehäuse 10 mit den Kammern 11, 12, der Lochblende 15 und den Stutzen 13, 14 bildet mithin eine Meßstrecke in der Form, daß die hindurchströmende Flüssigkeitsmenge ermittelt werden kann, wenn der Druck in den Kammern 11, 12 gemessen wird. Die Durchflußmenge errechnet sich nach der oben bereits erwähnten Formel :

Durchflußmenge = $(\pi \, \Delta \, PR^4)/(8 \, \gamma \, l)$,

wobei $\gamma$ die Viskosität des hindurchtretenden Fluids angibt.

Wie aus Fig. 2 hervorgeht, sind die Druckkammern 11, 12 an einer Seite über eine flache dünne Membran 20, 21 abgeschlossen. In Fig. 2 ist schematisch auch ein Meßwandler 30 dargestellt. Er besitzt ein starres Gehäuse 31, das im unteren Bereich einen Hohlraum 32 bildet, der nach unten durch eine Meßmembrane 33 abgeschlossen ist. Über einen Kanal 34 steht der Hohlraum 32 in Verbindung mit einem Drucksensor 35, dessen Wirkfläche (nicht gezeigt) dem Kanal 34 zugekehrt ist. Der Kanal 34 steht in Verbindung mit einer Verbindungsleitung 36, in der ein Absperrhahn 37 angeordnet ist. Die Verbindungsleitung 36 führt zu einem Ballon 38. Das gesamte System aus Hohlraum 32, den Kanälen 34, 36 und dem Ballon 36 ist mit einer Meßflüssigkeit gefüllt. Wird der Meßwandler 30 auf zum Beispiel die Membrane 21 der Druckkammer 12 gesetzt, verformt sich die zunächst unter Vorspannung nach außen wölbende Meßmembrane 33 zurück, so daß sie annähernd flach mit der Membrane 21 der Druckkammer 12 in Eingriff gelangt (siehe Fig. 3) Hierbei ist der Absperrhahn 37 geöffnet. Anschließend wird er abgesperrt, nachdem das durch das Zurückverformen der Meßmembrane 33 verdrängte Volumen in den Ausgleichsbehälter 38 gefördert wurde.

Die Auslenkung der Membranen 20, 21 der Druckkammern 11, 12 ist proportional zu dem Druck in den Kammern 11, 12. Diese Auslenkung überträgt sich auf die Meßmembrane 33, welche über die Meßflüssigkeit im Hohlraum 32 an die Wirkfläche des Sensors 35 angekoppelt ist. Auf diese Weise ist es möglich, ohne Kontakt mit dem Fluid in den Kammern 11, 12 eine sehr feine Durchflußmengenmessung vorzunehmen. Der Drucksensor kann von herkömmlicher Bauart sein, und seine Meßleitungen 39 führen zu einer geeigneten Auswerte- bzw. Steuervorrichtung, beispielsweise um eine Infusionspumpe zu regeln bzw. zu steuern und ggf. eine Anzeige der Durchflußmenge zu ermöglichen. Wie erkennbar, ist der der Druckkammer 11 zugeordnete Meßwandler nur angedeutet. Er kann in gleicher Weise aufgebaut sein wie der Meßwandler 30.

Es sei angemerkt, daß bereits sehr geringe Verformungen der Membranen 20, 21 bzw. 33 zu Meßausschlägen führen. Plötzlich auftauchende höhere Drücke sind unschädlich, weil sie durch den Meßwandler 30 gedämpft werden.

Fig. 4 zeigt ein Gehäuse 40 ähnlich dem Gehäuse 10 nach den Figuren 1 bis 3, in dem drei Druckkammern 41, 42, 43 angeordnet sind, die über Lochblenden 44 bzw. 45 miteinander verbunden sind. Sie bilden wiederum eine Meßstrecke entsprechend der oben beschriebenen Anordnung. Aufgrund der drei Druckkammern 41, 42, 43, die hier in vertikaler Anordnung gezeigt sind, läßt sich eine Druckdifferenz zwischen den Kammern 41, 42; 42, 43 und 41, 43 ermitteln, so daß eine Fehlfunktion eines Meßwandlers oder der Auswerteelektronik oder dergleichen ohne weiteres durch einen Vergleich der Meßwerte festgestellt werden kann. Wenn die Druckdifferenzen: P42 - P41 = Δ P41, 42; P43 - P42 = Δ P 43, 42 und P43 - P41 = Δ P43, 41 sind, muß folgende Voraussetzung gegeben sein, wenn ein vorgegebener Durchflußwert eingehalten werden soll :

$$\Delta P43, 41 = \Delta P41, 42 + \Delta P43, 42.$$

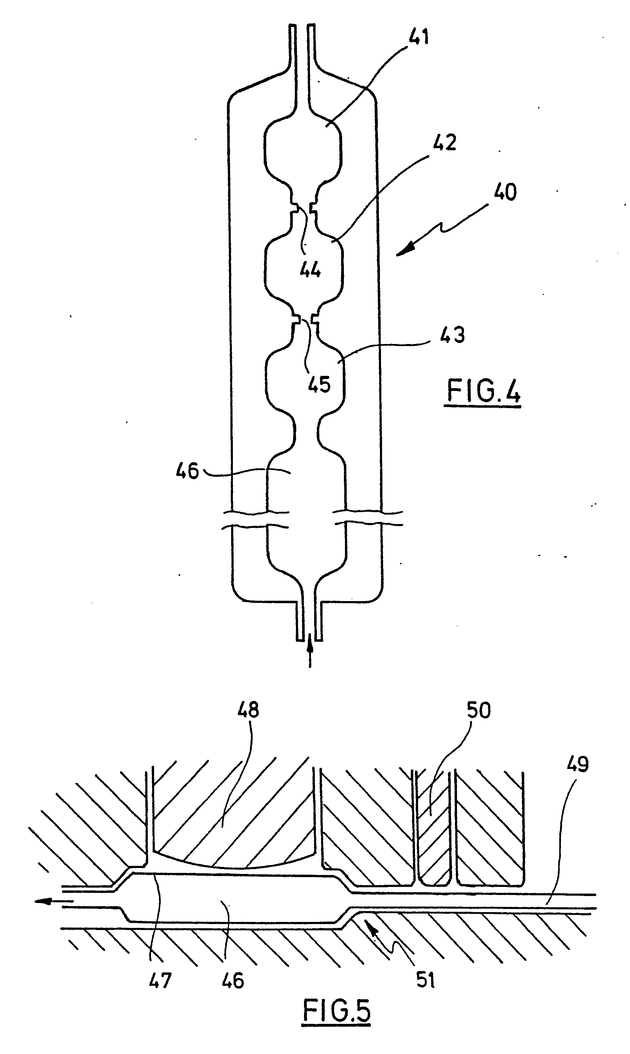

Wie aus Fig. 4 zu erkennen, ist im Gehäuse 40 auch noch eine Vorkammer 46 angeordnet, die in Strömungsrichtung vor der ersten Druckkammer 43 liegt. Ihr Volumen ist so groß, daß es mindestens gleich dem Volumen der drei Druckkammern 41, 42, 43 und dem nachgeschalteten Leitungssystem, beispielsweise beim Infusionsbesteck, ist. Sinn der Vorkammer 46 ist, das Einbringen von Fluid bzw. Flüssigkeit in die Druckkammern und die nachgeschaltete Leitung zu erleichtern. Die verhältnismäßig großvolumige Vorkammer 46 kann beispielsweise durch Schwerkraft eingefüllt werden. Wie aus Fig. 5 zu erkennen, ist die Vorkammer 46 mit einem nachgebenden Wandabschnitt 47 versehen, dem ein Kolben 48 zugeordnet ist. Der Kolben kann von einer mechanischen, elektromagnetischen, hydraulischen oder pneumatischen Vorrichtung betätigt werden (nicht gezeigt). Der Einlaß der Vorkammer 46 ist mit einem Schlauch 49 verbunden, dem eine Schlauchklemme 50 zugeordnet ist, die ebenfalls durch eine geeignete Betätigung betätigbar ist. Die gezeigten Teile befinden sich, wie in Fig. 5 dargestellt, innerhalb eines geeigneten Gehäuses oder einer Halterung 51. Ist die Vorkammer 46 zum Beispiel mit einer Infusionsflüssigkeit gefüllt, wird die Schlauchklemme 50 betätigt und sperrt somit die Vorkammer 46 von der Eingangsseite her ab. Anschließend wird der Kolben 48 betätigt, der dadurch die Flüssigkeit aus der Vorkammer 46 herausdrückt und in die Druckkammern 43, 42 und 41 hinein und in das nachfolgende Schlauchsystem. Auf diese Weise kann ein schnelleres Einfüllen von Infusionsflüssigkeit in die Druckkammern und das Schlauchsystem stattfinden als es sonst bei normaler Schwerkraftbefüllung der Fall wäre aufgrund der verhältnismäßig engen Blenden 44, 45.

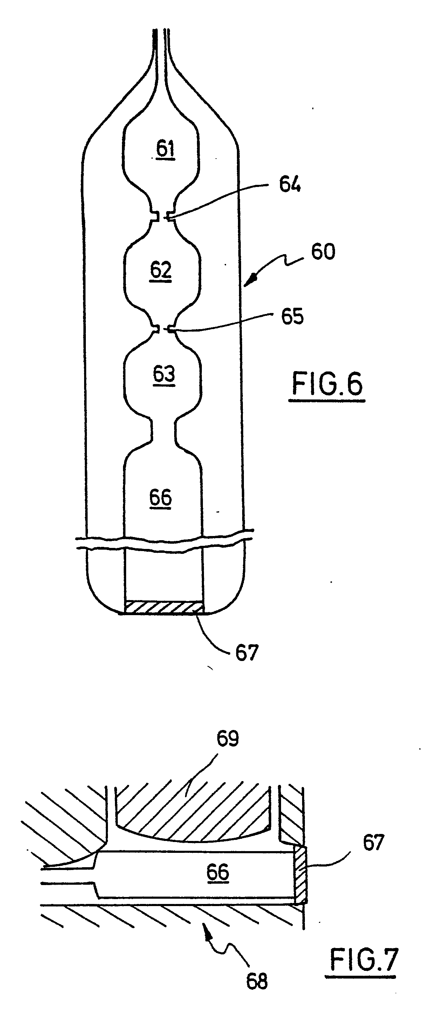

In Fig. 6 ist ein Gehäuse 60 dargestellt, das im wesentlichen dem Gehäuse 40 nach Fig. 4 gleicht. Es enthält Druckkammern 61, 62, 63, die durch Lochblenden 64 bzw. 65 miteinander verbunden sind. Auch eine Vorkammer 66 ist vorgesehen, entsprechend der Vorkammer 46 nach Fig. 4. Da insoweit Übereinstimmung mit der Ausführungsform nach Fig. 4 besteht, wird hierauf im einzelnen nicht mehr eingegangen. Es versteht sich, daß den Druckkammern gemäß der Ausführungsform nach Fig. 4 bzw. nach Fig. 6 Druckmeßwandler zugeordnet werden können, wie sie zum Beispiel in Fig. 2 dargestellt sind. Abweichend gegenüber Fig. 4 ist die Vorkammer 66 durch ein Septum 67 abgeschlossen, beispielsweise eine elastomere Scheibe, die mit der Nadel einer Injektionsspritze ohne weiteres durchstochen werden kann. Das Gehäuse 60 ist wiederum in einer geeigneten Halterung 68 angeordnet, die auch einen Kolben 69 aufweist, der entsprechend dem Kolben 48 nach Fig. 5 ausgebildet und betätigbar ist. Mit Hilfe einer Injektionsspritze kann mithin die Vorkammer 66 mit Infusionsflüssigkeit gefüllt werden. Durch Betätigung des Kolbens 69 kann die Flüssigkeit in gewünschter Menge durch die Druckkammern 61 bis 63 und die anschließende Leitung gefördert werden, zum Beispiel zum Zumessen von Infusionsflüssigkeit in den menschlichen Körper. Mit Hilfe des Kolbens 69 läßt sich eine sehr feine Dosierung vornehmen. Durch die Druckmessung in der oben beschriebenen Weise kann dabei eine genaue Kontrolle der geförderten Flüssigkeit vorgenommen werden.

## Ansprüche

1. Durchflußmeßvorrichtung für Fluide mit einer Meßstrecke und einem Meßwandler zwischen einem Flüssigkeitszu- und -ablauf, dadurch gekennzeichnet, daß die Meßstrecke mindestens zwei Druckkammern (11, 12) aufweist, die jeweils über einen kalibrierten Durchgang (15) miteinander verbunden sind und jeder Druckkammer (11, 12) ein Meßwandler (30) zugeordnet ist zur Erzeugung eines dem Druck in der Druckkammer (11, 12) entsprechenden Meßsignals.

2. Vorrichtung nach Anspruch 1, dadurch gekennzeichnet,daß der Durchgang von einer Lochblende (15) gebildet ist.

3. Vorrichtung nach Anspruch 1, dadurch gekennzeichnet,daß ein Röhrchen den Durchgang bildet.

4. Vorrichtung nach einem der Ansprüche 1 bis 3, dadurch gekennzeichnet, daß die Druckkammern (11, 12) einen nachgebenden Wandabschnitt aufweisen und der Meßwandler (30) dem nachgebenden Wandabschnitt zugeordnet ist zur Messung der Auslenkung des Wandabschnitts.

5. Vorrichtung nach Anspruch 4, dadurch gekennzeichnet,daß der Wandabschnitt von einer vorzugsweise dünnen Membrane (20, 21) gebildet ist, die im unbelasteten Zustand im wesentlichen flach ist.

6. Vorrichtung nach Anspruch 5, dadurch gekennzeichnet,daß der Meßwandler (30) ebenfalls einen nachgiebigen Wandabschnitt, vorzugsweise eine Meßmembrane (33) aufweist, die mit der Membrane (20, 21) einer Druckkammer (11, 12) in 'Kontakt und die in Wirkkontakt mit der Ansprechfläche des Meßwandlers (30) bzw. einer Druckmeßvorrichtung bringbar ist.

7. Vorrichtung nach Anspruch 6, dadurch gekennzeichnet,daß die Meßmembrane (33) einen von unnachgiebigen Wänden gebildeten Hohlraum (32) des Meßwandlers (30) abschließt, dem die Ansprechfläche des Meßwandlers bzw. der Druckmeßvorrichtung (35) zugeordnet ist.

8. Vorrichtung nach Anspruch 7, dadurch gekennzeichnet,daß der Hohlraum (32) mit einer Meßflüssigkeit gefüllt ist.

9. Vorrichtung nach Anspruch 8, dadurch gekennzeichnet,daß der Hohlraum (32) über eine einen Absperrhahn (37) auf weisende Leitung (36) mit einem Ausgleichsbehälter (38) verbunden ist, der vorzugsweise als elastischer Ballon ausgebildet ist.

10. Vorrichtung nach einem der Ansprüche 6 bis 9, dadurch gekennzeichnet, daß die Meßmembrane (33) bei Nichtkontakt mit dem nachgebenden Wandabschnitt (20, 21) der Druckkammer (11,12) unter Spannung nach außen gewölbt ist.

11. Vorrichtung nach einem der Ansprüche 6 bis 10, dadurch gekennzeichnet, daß die Membranen (20, 21) aus dünnem elastischem Material bestehen und aufgrund einer Vorspannung vollständig flächig in Kontakt sind.

12. Vorrichtung nach einem der Ansprüche 1 bis 11, dadurch gekennzeichnet, daß den Druckkammern (11,12) getrennte Meßwandler zugeordnet sind.

13. Vorrichtung nach einem der Ansprüche 1 bis 12, gekennzeichnet durch eine Ausbildung der Druckkammern (11, 12), insbesondere des dem Auslaß zugeordneten Wandbereichs der Druckkammern, daß bei einem horizontalen oder vertikalen Strömungsverlauf keine Blasenansammlung stattfindet.

14. Vorrichtung nach Anspruch 6,dadurch gekennzeichnet,daß die Membrane aus dünnem, elastischem sich piezoelektrisch verhaltendem Material besteht zur Erzeugung eines der Auslenkung der Membran entsprechenden Signals.

15. Vorrichtung nach einem der Ansprüche 1 bis 14, dadurch gekennzeichnet, daß die Druckmeßkammern (11, 12) und die Durchgänge in einem gemeinsamen Gehäuse (10) geformt sind.

16. Vorrichtung nach einem der Ansprüche 1 bis 15, dadurch gekennzeichnet, daß der in Strömungsrichtung gesehen ersten Druckkammer (43) eine Vorkammer (46) vorgeordnet ist, deren Volumen größer ist als das gesamte Volumen der Druckkammern (41, 42, 43) und der nachgeschalteten Ausgangsleitung hinter der letzten Druckkammer (41), dem Einlaß der Vorkammer (46) eine Verschlußvorrichtung (50) und der Vorkammer (46) eine das Arbeitsvolumen der Vorkammer (46) verkleinernde Pumpvorrichtung (48) zugeordnet sind.

17. Vorrichtung nach Anspruch 16, dadurch gekennzeichnet, daß die Verschlußvorrichtung eine Schlauchklemme (50) aufweist.

18. Vorrichtung nach Anspruch 16 oder 17, dadurch gekennzeichnet, daß zumindest ein Wandabschnitt der Vorkammer (46) nachgebend ausgebildet ist und der dem nachgebenden Wandabschnitt ein von einer Betätigungsvorrichtung betätigbarer Kolben zugeordnet ist.

19. Vorrichtung nach Anspruch 18, dadurch gekennzeichnet, daß der Kolben (48) Bestandteil einer Mikropumpe ist.

20. Vorrichtung nach einem der Ansprüche 16 bis 19, dadurch gekennzeichnet, daß die Vorkammer (66) durch ein Septum (67) verschlossen ist.

FIG.1

FIG.2

FIG.3

FIG.4

FIG.5

FIG.6

FIG.7